# EUROPEAN PATENT SPECIFICATION

(11) **EP 4 558 230 B1**
(45) Date of publication and mention of the grant of the patent: **01.04.2026**
(21) Application number: 23749172.5
(22) Date of filing: 20.07.2023
(51) Int. Cl.: A63B 21/00, A63B 22/06, A63B 71/06, A63B 24/00, A63B 69/16

(54) **SYSTEM FOR PHYSICAL ACTIVITY, AND RELATED METHOD**
SYSTEM FÜR PHYSISCHE AKTIVITÄT UND ZUGEHÖRIGES VERFAHREN
SYSTÈME D'ACTIVITÉ PHYSIQUE ET PROCÉDÉ ASSOCIÉ

(30) Priority: 22.07.2022 IT 202200015501
(43) Date of publication of application: 28.05.2025
(73) Proprietor: Università Vita-Salute San Raffaele, 20132 Milano (IT); Fondazione Cariplo, 20121 Milano (MI) (IT)
(72) Inventor: DE' SPERATI, Claudio, 20132 Milano (MI) (IT)
(74) Representative: Buzzi, Notaro & Antonielli d'Oulx S.p.A.
(86) International application number: PCT/IB2023/057415
(87) International publication number: WO 2024/018419

(56) References cited:
- EP-A1- 1 683 550
- EP-A1- 4 035 747
- US-A1- 2013 165 195
- US-A1- 2014 135 173
- US-A1- 2016 375 308

## Description

### Technical field

The embodiments of the present disclosure relate to solutions for carrying out sports activities.

### Background

A great number of sports equipment is known for carrying out physical activity, in particular in a gym or home environment. For example, one type of sports equipment is the exercise bike or stationary bike, which makes it possible to simulate the use of a bicycle, such as for cardiovascular training of the physique as in the real use of it. Typically, a stationary bike does not have wheels, but includes one or more actuators to set/adjust manually or by means of a control unit the pedaling resistance/intensity. For example, these actuators may include mechanical, magnetic or electromagnetic actuators.

Recently, solutions have been proposed in which virtual reality (VR) is used in conjunction with exercise on the stationary bike. For example, in addition to the visualization of a virtual environment of a route, a control unit of the stationary bike may vary the resistance applied to the pedals of the stationary bike according to the visualized route (uphill, downhill, etc.). For example, to visualize the virtual environment, the system may include one or more screens that project images of landscapes during the exercise on the stationary bike, where these one or more screens are placed e.g., on the handlebar of the stationary bike or in front of the stationary bike, or are integrated into a virtual reality viewer that allows the user to immerse himself or herself in a simulated 3D landscapes while pedaling on the exercise bike.

Such solutions are thus able to enhance the user experience and stimulate the performance of physical exercises. For example, these solutions are particularly useful for people with mobility impairments, such as elderly people, who can perform physical exercises in this way in an indoor environment, such as an assisted living facility, simulating the experience of an outdoor ride. For example, in this context may be cited documents US 11,144,116 B2, WO 2001/024892 A2, WO 2019/112202 A1 or J. R. Bruun-Pedersen, K. S. Pedersen, S. Serafin and L. B. Kofoed, "Augmented exercise biking with virtual environments for elderly users: A preliminary study for retirement home physical therapy", 2nd Workshop on Virtual and Augmented Assistive Technology (VAAT), 2014, pp. 23-27, doi:

More advanced solutions for interacting with the virtual environment have also been proposed in order to enhance the user experience. For example, other subjects and/or avatars may be shown in the virtual environment cycling together with the user to stimulate competition or collaboration. In this context may be cited document E. R. Høeg, J. R. Bruun-Pedersen, S. Cheary, L. K. Andersen, R. Paisa, S. Serafin, B. Lange, "Buddy biking: a user study on social collaboration in a virtual reality exergame for rehabilitation", Virtual Reality, July 2021, pp. 1-18, doi: 10.1007/s10055-021-00544-z. In addition, the user's movement may be synchronized with the virtual environment, for example, to simulate the orientation/tilt of the user's head, changing the perspective within the virtual environment. Similarly, additional actuators can create effects in the user's environment depending on the path shown in the virtual environment, e.g., tilting the stationary bike, creating acoustic, visual or haptic effects, generating with a fan an airflow proportional to the user's speed in the virtual environment, etc. Furthermore, special solutions can be implemented to facilitate visual exploration during virtual biking, such as augmented gaze. In this context may be cited document de'Sperati C, Dalmasso V, Moretti M, Høeg ER, Baud-Bovy G, Cozzi R, Ippolito J. Enhancing Visual Exploration through Augmented Gaze: High Acceptance of Immersive Virtual Biking by Oldest Olds. Int J Environ Res Public Health. 2023 Jan 17;20(3):1671. doi: 10.3390/ijerph20031671. PMID: 36767037; PMCID: PMC9914324.

Document EP 1683550 A1 discloses a system for connecting a stationary bicycle with an outdoor bicycle via a communication network, wherein only the stationary bicycle's resistance is adjusted based on the outdoor conditions.

### Summary of the invention

The invention is defined by the appended claims.

### Outline of the disclosure

The present disclosure relates to solutions, which permit to further increase the experience of pedaling a real path, specifically to enable a first user to pedal a stationary bike by virtually replicating the experience of a second user simultaneously pedaling a bicycle in an outdoor environment.

According to one or more embodiments, this object is achieved via a system having the distinctive elements set forth specifically in the claims that follow. The embodiments also concern a related method.

The claims are an integral part of the technical teaching of the description provided here.

As mentioned before, various embodiments of the present disclosure relate to a system for performing physical activity. In various embodiments, the system comprises a stationary bike comprising an actuator configured to set a pedaling resistance of the stationary bike and a bicycle (or similar vehicle) comprising at least two wheels.

In various embodiments, the stationary bike includes a first communication interface and the bicycle including a second communication interface, wherein the first communication interface and the second communication interface are configured to exchange data via a Wide-Area Network.

In various embodiments, the system enables audio interaction between the users. For this purpose, the stationary bike includes a first microphone configured to record the voice of a first user using the stationary bike, a first speaker and a first processing circuit. In a complementary manner, the bicycle includes a second microphone configured to record the voice of a second user using the bicycle, a second speaker, and a second processing circuit. For example, in various embodiments, the first processing circuit records a first audio signal via the first microphone and sends the first audio signal via the first communication interface to the second communication interface. The second processing circuit then receives the first audio signal via the second communication interface from the first communication interface and reproduces the first audio signal via the second speaker. Similarly, the second processing circuit records a second audio signal via the second microphone and sends the second audio signal via the second communication interface to the first communication interface. The first processing circuit then receives the second audio signal via the first communication interface from the second communication interface and reproduces the second audio signal via the first speaker.

In various embodiments, the system also shows the path followed by the bicycle. For this purpose, the bicycle includes a camera configured to film a path being followed by the bicycle, and the stationary bike includes a screen. For example, in various embodiments, the second processing circuit records an image sequence via the camera and sends the image sequence via the second communication interface to the first communication interface. The first processing circuit then receives the image sequence via the first communication interface from the second communication interface and displays the first image sequence via the screen.

In various embodiments, the viewpoint of the image sequence can be changed. For example, for this purpose, the stationary bike includes a position sensor configured to detect the position of the first user's head and/or eyes. In this case, the camera may be a camera configured to capture panoramic images or the camera includes a plurality of cameras configured to capture a plurality of images with different orientations with respect to the bicycle. In this case, the first processing circuit and the second processing circuit may record via the camera a sequence of the panoramic images or the plurality of images with different orientations, and generate the image sequence as a function of the position and the sequence of the panoramic images or the plurality of images with different orientations. Alternatively, the camera may be a rotating camera, and the second control circuit may rotate the camera as a function of the position.

In various embodiments, the stationary bike may also include a camera and the bicycle may include a screen. In this case, the first processing circuit may record an additional sequence of images via the second camera. In various embodiments, such a sequence is not shown directly, but the first processing circuit and/or the second processing circuit generate an avatar of the first user's face as a function of the image sequence, and the second processing circuit shows the avatar by means of the screen, and/or drives the actuators of a robotic head as a function of the image sequence.

Additionally or alternatively, the system permits an interaction between the stationary bike and the bicycle.

For example, in various embodiments, the bicycle includes a sensor configured to detect first data indicative of the effort that the second user generates or should generate, for example by determining the speed of the bicycle, the torque applied to the bicycle pedals, and/or the tilt of the bicycle. In this case, the second processing circuit may send the first data to the first processing circuit, and the first processing circuit may drive the actuator to vary the pedaling resistance of the stationary bike as a function of the first data, such that the effort of the first user increases when the effort of the second user increases and the effort of the first user decreases when the effort of the second user decreases.

Additionally or alternatively, the bicycle may be a pedal-assisted electric bike, and the stationary bike may include a second sensor configured to detect second data indicative of the rotation speed of the pedals of the stationary bike and/or the torque applied to the pedals of the stationary bike. In this case, the first processing circuit may send the second data to the second processing circuit, and the second processing circuit may vary the level of assistance provided by the pedal-assisted electric bike as a function of the second data, such that the level of assistance is increased when the pedal rotation speed and/or the torque applied to the pedals is increased, and the level of assistance is decreased when the pedal rotation speed and/or the torque applied to the pedals is decreased. Alternatively, the second processing circuit may directly vary the bicycle speed as a function of the pedal rotation speed of the stationary bike.

In various embodiments, the stationary bike may also include a sensor to detect the actuating state of a lever and/or the force applied to the lever. In this case, the first processing circuit may send the actuation state of the lever and/or the force applied to the lever to the second processing circuit, and the second processing circuit is configured to vary the level of assistance provided by the pedal-assisted electric bike as a function of the actuation state of the lever and/or the force applied to the lever, such that the level of assistance is reduced when the lever is actuated. Alternatively, the second processing circuit may directly brake the bicycle as a function of the actuation state of the lever and/or the force applied to the lever.

Additionally or alternatively, the bicycle may include a handlebar having associated a servo steering, and the stationary bike may include a third sensor configured to detect third data indicative of the rotation of a handlebar of the stationary bike. In this case, the first processing circuit may send the third data to the second processing circuit, and the second processing circuit may drive the servo-steering as a function of the third data, e.g., by applying a torque to the bicycle handlebar that is proportional to the rotation of the stationary bike handlebar. Alternatively, the second processing circuit may directly turn the bicycle handlebar as a function of the rotation of the stationary bike handlebar.

Accordingly, the solutions disclosed here permit to increase the experience of riding in the world realistically and with companionship for those who cannot.

### Brief description of the drawings

The embodiments of the present description will now be described with reference to the attached drawings, which are provided as non-limiting examples only, and in which:
- Figure 1 shows an embodiment of a system for performing physical activity in accordance with the present disclosure;
- Figure 2 shows an embodiment of a stationary bike adapted to be used in the system of Figure 1;
- Figure 3 shows an embodiment of a bicycle, such as a pedal-assisted bike, adapted to be used in the system of Figure 1;
- Figure 4 shows a first embodiment of a control circuit for the pedal-assisted bike of Figure 3;
- Figure 5 shows a second embodiment of a control circuit for the pedal-assisted bike of Figure 3;
- Figure 6 shows a first embodiment of an audio and/or video streaming system for the stationary bike of Figure 2;
- Figure 7 shows a second embodiment of an audio and/or video streaming system for the stationary bike of Figure 2;
- Figure 8 shows a first embodiment of an audio and/or video streaming system for the bicycle of Figure 3; and
- Figure 9 shows a second embodiment of an audio and/or video streaming system for the bicycle of Figure 3.

### Detailed description

In the following description, numerous specific details are given to provide a thorough understanding of the embodiments. Embodiments may be implemented without one or several specific details, or with other methods, components, materials, etc. In other cases, well-known operations, materials, or structures are not represented or described in detail so as to avoid making aspects of the embodiments unclear.

A reference throughout the present description to "an embodiment" means that a particular feature, distinctive element or structure described with reference to the embodiment is included in at least one embodiment. Thus, the use of the phrase "in an embodiment" in the various parts of the present description do not necessarily all refer to the same embodiment. Also, the particular features, distinctive elements or structures may be combined in any way, in one or more embodiments.

The references used herein are provided only for convenience and hence do not define the sphere of protection or the scope of the embodiments.

As mentioned earlier, the present description provides solutions to enhance the experience of a user using an exercise bike or stationary bike.

Older people living in facilities or at home alone frequently suffer from three major problems: cloistered living, declining physical activity and affective depletion. The solutions proposed here address these problems, allowing the experience of cycling through the world realistically and with companionship even for those who cannot.

As explained before, virtual pedaling systems are known, where a user can pedal on an electric stationary bike in front of a screen that displays a virtual route synchronized with the pedaling. In the solutions described here, this approach is extended to a real-time interaction with a remote bicycle that follows a real path, allowing an interaction with the remote bicycle user and/or an interaction between the stationary bike and the remote bicycle.

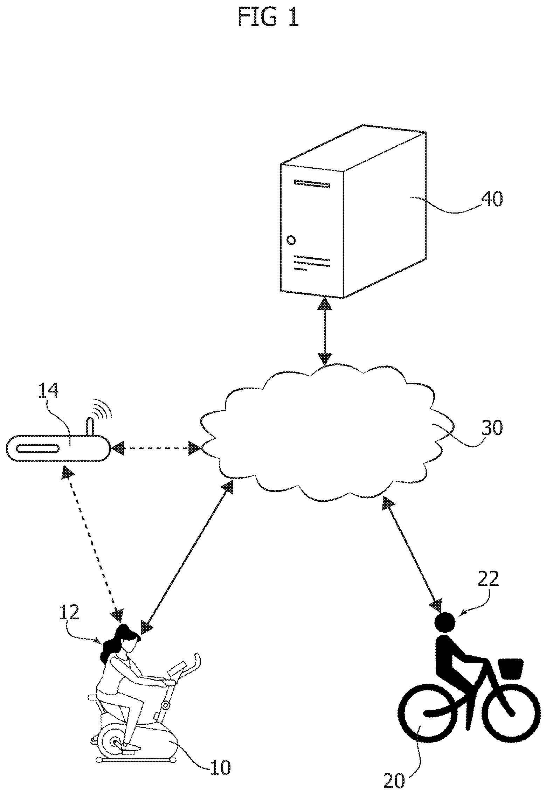

Figure 1 schematically shows a system for performing physical activity according to the present description. In the embodiment considered, a first user 12, such as an elderly person, uses a stationary bike 10. Conversely, a second user 22, such as a relative of the first user 12, uses a bicycle 20 to follow a path in the real world.

In the embodiment considered, the stationary bike 10 and the bicycle 20 are configured to exchange data via a network 30, preferably a Wide Area Network (WAN), such as the Internet. For example, for this purpose, the bicycle 20 may include one or more transceivers for a mobile communication network, enabling the bicycle 20 to be connected to the network 30. Similarly, the stationary bike 10 may also include one or more transceivers for a mobile communication network. However, since the stationary bike 10 is static, other wired or wireless transceivers may also be used to connect the stationary bike 10 to the network 30. For example, Figure 1 schematically shows a router 14 that permits to connect the stationary bike 10 to the network 30 by using a Wi-Fi network, i.e., a network based on the IEEE 802.11 standards, or a wired connection via Ethernet. Preferably, the stationary bike 10 and the bicycle 20 exchange data by using the Internet Protocol (IP), for example, by using the Transmission Control Protocol (TCP) and/or the User Datagram Protocol (UDP).

In various embodiments, the connection between the stationary bike 10 and the bicycle 20 can also be managed at least in part via a server 40. For example, the server 40 may be used to authenticate the stationary bike 10 and the bicycle 20. For example, for this purpose, each stationary bike 10 and each bicycle 20 may have associated a univocal code and may be registered with the server 40. In various embodiments, all communication between the stationary bike 10 and the bicycle 20 may pass through the server 40. Alternatively, once authenticated, the server may pass data identifying the IP address of the stationary bike 10 to the stationary bike 20, and/or data identifying the IP address of the stationary bike 10 to the stationary bike 20, such that the stationary bike 10 and the bicycle 20 may exchange (at least in part) data directly. For example, these solutions are commonly used in the context of the so-called Internet-of-Things (IoT), where the server 20 may provide an access token, such as an OAuth token, e.g., OAuth 2.0, which enables access to the stationary bike 20 or the stationary bike 10.

Specifically, in various embodiments, the connection between the stationary bike 10 and the bicycle 20 is used for a two-way communication that permits an interaction between the users 12 and 22.

For example, in various embodiments, such communication includes an audio link, which allows the user 12 to converse with the user 22 who is riding bicycle 20.

In various embodiments, the bicycle 20 also includes one or more cameras, and the bicycle 20 sends a video stream of the environment in which the bicycle 20 is located. Thus, the stationary bike 10 may include one or more screens for displaying the video stream. Accordingly, while in the known solutions the route is recorded in advance and the recorded route is then reproduced by synchronizing the display with the operation of the stationary bike, in the embodiment described herein, the video streaming may occur in real time, i.e., the user 12 can observe in real time the route that the bicycle 20 follows, i.e., based on the forward speed of the bicycle 20.

As will be described in more detail in the following, such a video stream may be captured, e.g., by a static camera, i.e., the camera position is fixed/static with respect to the bicycle 20. Alternatively, the camera may be rotatable as a function of instructions received from the stationary bike 10, i.e., the camera position is variable with respect to the bicycle 20. For example, the rotation instructions may be generated as a function of the position of the head of the user 12, which allows the camera to be rotated relative to the bicycle 20 with a rotation angle determined as a function of the position of the head of the user 12 relative to the stationary bike 10. Therefore, in this way, the user 12 can remotely control the position of the camera installed on the bicycle 20. Finally, the camera(s) may record panoramic images with a wide angle, for example 180° or 360°, and the stationary bike 10 may show a virtual perspective of the acquired environment in real time, by using for this purpose a so-called virtual viewpoint. For example, in this context may be cited document EP 2 150 065 B1, the content of which is incorporated herein by reference.

In various embodiments, the stationary bike 10 may also include one or more cameras configured to capture images, e.g., of the face of the user 12. Therefore, the bicycle 20 may include one or more screens to display such a video stream. Alternatively, the bicycle 20 may also only display an avatar of the face of the user. In various embodiments, such an avatar may also be dynamically generated by synchronizing the facial movement of the avatar with the facial movement of the user 12.

In various embodiments, the stationary bike 10 and the remote bicycle 20 may also exchange status data of the stationary bike 10 and/or the remote bicycle 20. For example, in this way, the resistance generated by an actuator of the stationary bike 10 may be set as a function of the tilt of the remote bicycle 20. Similarly, the bicycle 20 may be an electric bike, and the pedaling speed of the user 10 (or similar data) may be used to increase or decrease the pedaling assistance provided by the electric bike. Other data that can be exchanged are, for example, geolocation signals.

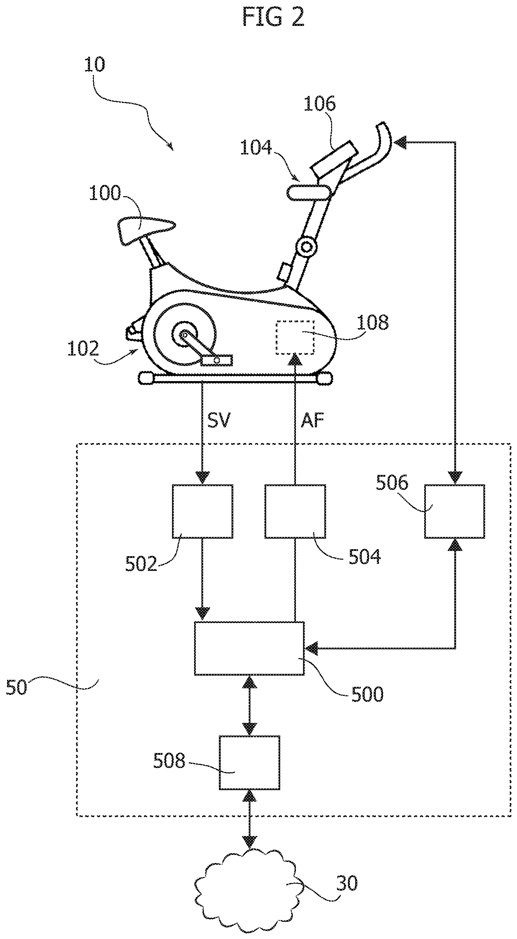

Figure 2 shows an embodiment of a stationary bike 10. In the embodiment considered, the stationary bike 10 includes pedals 102 that can be operated by a user 12. Typically, the stationary bike 10 also includes a saddle 100 and the user 12 can pedal via the pedals 102 when the user 12 is sitting on the saddle 100. In this case, the stationary bike 10 typically also includes a handlebar 104. In various embodiments, the pedals 102 may also be used for a so-called handlebar pedaling. An embodiment of stationary bike 10 may also involve that the saddle 100 and pedals 102 are physically separate and functionally independent components.

In the embodiment considered, the stationary bike 10 also includes an actuator 108 configured to set/adjust the pedaling resistance. For example, the actuator 108 may include one or more mechanical, magnetic, and/or electromagnetic actuators. In the embodiment considered, the stationary bike 10 thus includes a control circuit 50 configured to drive the actuator 108.

For example, in the embodiment considered, the control circuit 50 includes a driver circuit 502 configured to generate a control signal AF that sets the force/resistance to be applied via the actuator 108, and a processing circuit 500 configured to drive the driver circuit 502. For example, the processing circuit 500 may comprise an analog and/or digital processing circuit. For example, in various embodiments, the processing circuit 500 includes one or more microprocessors programmed via software code.

In various embodiments, the control circuit 50 further comprise an interface circuit 506 for communicating with a user interface 106, for example, to display a menu, to select a program, etc. For example, the user interface 106 is typically mounted on the handlebar 104 and may include a screen and buttons, and/or a touch screen.

In known stationary bikes, the processing circuit 500 then drives the actuator 108 via a circuit 504 to set a given resistance based on a predetermined profile, e.g., selected via the user interface 106.

Conversely, in various embodiments, the control circuit 50 includes a communication interface 508 to communicate via the network 30 and possibly the server 40 with the remote bicycle 20. For example, the communication interface 508 may be a communication interface of a mobile radio network, such as a GPRS (General Packet Radio Service), UMTS (Universal Mobile Telecommunications System), HSPA (High-Speed Packet Access) or LTE (Long Term Evolution) modem, etc., in which the communication interface 508 is configured to send data to the WAN 30 via a base station. However, the communication interface 508 may also include other one or more communication interfaces for communicating with the network 30, such as an Ethernet communication interface, a WiFi communication interface in accordance with one of the versions of IEEE 802.11 and/or WiMAX (Worldwide Interoperability for Microwave Access) standard, wherein the interface is configured to send data to the network 30 via an access point.

Accordingly, in various embodiments, the processing circuit 500 is configured to set the resistance applied by the actuator 108 (exclusively) as a function of data received from the remote bicycle 20. Alternatively, the processing circuit 500 may also set the resistance exclusively based on data set via the user interface 106, or jointly based on data received from the remote bicycle 20 and data set via the user interface 106. For example, in various embodiments, the processing circuit 500 may support at least two modes of operation, e.g., a first mode in which data set via the user interface 106 are used exclusively (as in a conventional stationary bike), and in a second mode of operation data received from the remote bicycle 20 and data set via the user interface 106 may be used jointly. For example, the data set via the user interface 106 may indicate how much the feedback received from the bicycle 20 affects the resistance generated by the actuator 108.

For example, in various embodiments, the remote bicycle 20 may send data indicating the tilt of the stationary bike 20 or other data indicating the effort that the remote user 22 applies to the bicycle 20 or should apply to the bicycle 20. For example, the processing circuit 500 may be configured to drive the actuator 108 to increase the resistance when the data indicate that the remote bicycle 20 is uphill (or the user 22 applies more effort) and decrease the resistance when the data indicate that remote bicycle 20 is downhill (or the user 22 applies less effort). Thus, in this way the effort of the user 12 can be synchronized (at least partially) with the effort of the user 22.

In various embodiments, the control circuit 50 may include a sensor 502 configured to acquire a signal SV indicative of the pedaling speed and/or the effort applied to the pedals of the stationary bike 10. For example, in a conventional stationary bike the resistance, pedaling speed, and/or effort (or similar data such as torque or power) of the pedaling are displayed on the user interface 106. In various embodiments, the processing circuit 500 is configured to send one or more of these data to the remote bicycle 20 as well.

In various embodiments, the stationary bike 10 may also include additional sensors. For example, in various embodiments, the handlebar 104 of the stationary bike 10 is rotatable, and the control circuit 50 includes a sensor to detect the orientation of the handlebar 104. Additionally or alternatively, the handlebar may include a lever (similar to the brake lever of a conventional bicycle), and the control circuit 50 may include a sensor to detect the actuation of the lever, and possibly the force applied to the lever. Therefore, in this case, the processing circuit 500 is configured to send the orientation of the handlebar 104 and/or the lever actuation status to the remote bicycle 20.

Therefore, in various embodiments, the control circuit 50 is configured to detect data indicating the operating state of the stationary bike 10, such as the pedaling speed, handlebar orientation 104, and lever actuation, and send one or more of these data to the remote bicycle 20. Additionally or alternatively, in various embodiments, the control circuit 50 is configured to receive data from the remote bicycle 20 that are used to set the resistance applied by the actuator 108.

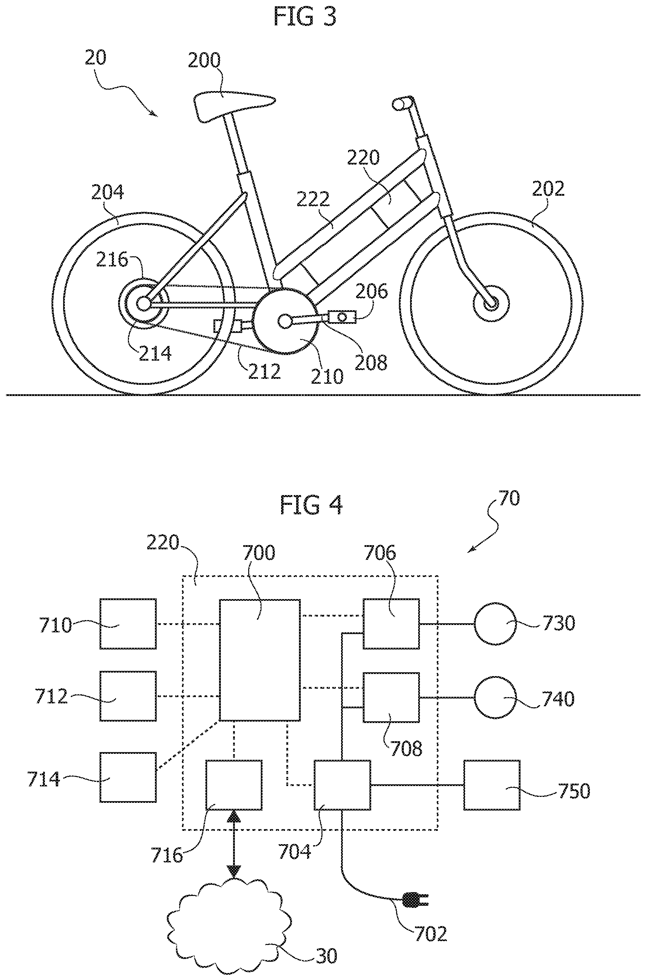

Figure 3 shows an embodiment of a bicycle 20. In the embodiment considered, the bicycle 20 includes a saddle 200, and at least two wheels, for example, a first wheel 202 at the front and at least one second wheel 204 at the rear. In the example considered, the bicycle 20 further includes the conventional arrangement of pedals 206 and crankset, comprising, for example, cranks 208 and one or more front chainrings 210. Typically, the front chainring 210 drives a rear sprocket 214 mounted coaxially with the rear wheel 204. For example, typically, the front chainring 210 is connected to a chain 212 that drives the rear sprocket 214.

In various embodiments, the bicycle 20 is an electric bicycle that operates in the electrically assisted pedaling mode, so-called e-bike. For example, in various embodiments, the rear sprocket 214 is not mounted rigidly on the hub 216 of the rear wheel 204 in order to directly drive that wheel. Instead, the rear sprocket 214 drives certain components of an electromechanical drive arrangement that are housed within the hub 216. Alternatively or additionally, an electromechanical drive arrangement may be provided in the hub of the front wheel 202 or may be configured to drive the front chainring 210.

As shown in Figure 4, the electromechanical transmission arrangement includes at least one electric motor 730 that is driven via a control circuit 70 that selectively connects the motor 730 to a battery 750, and preferably regulates the rotation speed of the motor 730 and/or the torque generated by the motor 730. Such electromechanical transmission arrangements are well known, for example from document US 2012/0012412 A1.

Figure 4 also shows an embodiment of the control circuit 70 for the electromechanical transmission arrangement. In the embodiment considered, the control circuit 70 includes a processing circuit 700, a battery management circuit 704, and a driver circuit 706 of the electric motor 730. In various embodiments, the processing circuit 700 is implemented with a microprocessor programmed via software code. Typically, the battery management circuit 704 is a bidirectional electronic converter that provides a regulated voltage to the driver circuit 706, while the driver circuit 706 generates one or more drive signals for the electric motor 730 as a function of one or more control signals received from the processing circuit 700, such as a signal indicative of a requested rotation speed or torque.

In various embodiments, to charge the battery 760, the battery management circuit 704 may be connected via a cable 702 to a mains outlet. In general, the electromechanical transmission arrangement may also include a generator 740 configured to produce electrical power. For this purpose, the generator 740 may be connected via a generator control circuit 708 to the battery management circuit 704, thereby charging the battery 730, for example, when the bicycle 20 is braked.

In various embodiments, the control circuit 70 may also include a user interface 712 consisting of, e.g., a screen and/or one or more indicators and/or one or more buttons.

As also shown in Figure 3, the components 700, 704, 706, and 708 may be located in a control casing/housing 220 mounted on the bicycle frame 20. The battery 730 may be located within an additional battery housing 222 also attached to the frame.

In various embodiments, to implement the pedal assist, the control circuit 70 may include one or more sensors 710. For example, a sensor 710 may detect the effort/torque applied to the pedals 206 and the processing circuit may increase the torque generated by the motor 730 when the effort applied to the pedals 206 increases, and decrease the torque generated by the motor 730 when the effort applied to the pedals 206 decreases.

In various embodiments, the electric bicycle 20 may include a lever, handpiece, swivel handle, or other types of controls 714, e.g., mounted on the handlebar, to set a level of contribution/assistance to be generated by the electric motor 730. In this case, the processing circuit 700 may be configured to increase or decrease the proportionality, or generally the ratio, between the torque generated by the motor 730 and the effort of the user 22 as a function of the input received from the controls 714. The person skilled in the art will appreciate that many solutions are known to control the motor speed or torque generated by the motor 730 as a function of one or more signals provided by the sensors 710, such as signals indicating the speed of rotation of the pedals, the torque applied to the pedals, and/or the tilt of the bicycle 20. These solutions have in common that an actuator, i.e., a continuous or discrete sensor, permits to set data indicating the level of assistance to be provided by the electric motor 730.

As mentioned earlier, in various embodiments, the control circuit 70 also includes a communication interface 716, such as a mobile network communication interface. In this case, regardless of whether the bicycle is electric or conventional, the processing circuit 700 may be configured to send one or more data sensed by the sensors 710 to the stationary bike 10. For example, the processing circuit 700 may send data indicating the torque applied to the pedals 206 of the bicycle 20, or other data indicative of the effort the user 22 is generating (e.g., with assistance) or should be generating (e.g., without assistance), e.g., data indicative of the tilt of the bicycle 20 and possibly the speed of the bicycle 20. In particular, as mentioned before, the stationary bike 10 may use these data to drive the actuator 108. In general, instead of directly monitoring the tilt and/or speed of the bicycle 20, the sensors 710 may include a position sensor, preferably a satellite navigation receiver, such as a GPS, GALILEO, and/or GLONASS receiver. In fact, by periodically sensing the position of the bicycle 20, the processing circuit 700 and/or the processing circuit 500 may calculate the distance traveled by the bicycle 20 and thus the speed of bicycle 20. Similarly, based on changes in consecutive positions in space, the processing circuit 700 and/or the processing circuit 500 may estimate the inclination of bicycle 20. Alternatively, the processing circuit 700 and/or the processing circuit 500 may access a map database (e.g., maintained by the server 40) that includes information for determining the path inclination for a given position of the bicycle 20.

Additionally or alternatively, in various embodiments, the processing circuit 700 may receive via the communication interface 716 the rotation speed and/or effort applied to the pedals of the stationary bike 10 and (in the case of an electric bicycle) adapt the assistance level of the electric bicycle 20. For example, the processing circuit 700 may increase the assistance level, e.g., increase the torque generated by the motor 730, when the received data indicate that the rotation speed and/or the effort applied to the pedals of the stationary bike 10 increase, and decrease the assistance level, e.g., decrease the torque generated by the motor 730, when the received data indicate that the rotation speed and/or the effort applied to the pedals of the stationary bike 10 decrease. For example, in various embodiments, the processing circuit 700 is configured to vary, e.g., scale, the signal provided by the controls 714 as a function of the data provided by the stationary bike 10.

As mentioned before, in various embodiments, the stationary bike 10 may also include additional sensors. For example, in various embodiments, the stationary bike 10 includes a lever, e.g., similar to the lever of a bicycle brake, and the processing unit 500 may send data indicating the actuation state of the lever and possibly the force applied to the lever. In this case, the processing circuit 700 may then receive these data and reduce the level of assistance, e.g., reduce the torque generated by the motor 730, when the received data indicate that the lever has been actuated, e.g., by reducing the level of assistance in proportion to the force applied to the lever, thus simulating a remote braking.

Alternatively or additionally, the handlebar 104 of the stationary bike 10 may be rotatable, and the control circuit 50 includes a sensor to detect the orientation of the handlebar 104. In this case, the bicycle handlebar 20 may be implemented with a servo-steering, i.e., an additional motor 730 that applies torque to the handlebar, and the processing circuit 700 may vary the torque applied by the motor to the handlebar, depending on the orientation of the stationary bike handlebar 104. For example, in this context may be cited document CN 203111409 U. Specifically, this document describes a servo steering for a bicycle, for example, a pedal-assisted bicycle.

The inventor has observed that this additional torque applied to the handlebar of the bicycle 20 may generate instability for the balance of the bicycle 20. This instability may be reduced by using other pedal-operated vehicles 206 (with or without a pedal assist) instead of the bicycle 20, wherein such a vehicle includes more than two wheels, such as a tricycle with two rear wheels 204 or two front wheels 202. Alternatively, the bicycle 20 may include a pair of stabilizing wheels on the sides of the rear wheel 204 of the bicycle 20. In general, such stabilizing wheels may be installed in a fixed or removable manner.

Additionally, in various embodiments, the stabilizing wheels may be activated in a motorized manner. For example, for this purpose, the stabilizing wheels may be mounted on a support, and the bicycle 20 may include an additional motor 730 to move the stabilizing wheels to a first position where the stabilizing wheels are inactive (i.e., not in contact with the ground) and a second position where the stabilizing wheels are operational (i.e., in contact with the ground). In general, the displacement between the two positions may occur by any longitudinal displacement and/or a rotation. For example, in this context may be cited documents US 9,096,285 B2 and US 9,771,029 B2.

For example, in various embodiments, the processing circuit 700 may be configured to receive one or more signals via the user interface 712 and, in response to such signal(s), drive the actuator in such a way as to move the stabilizing wheels from the first position to the second position (or vice versa). Alternatively or additionally, the processing circuit 700 may monitor signals received from the stationary bike 10 and/or roll data of the bicycle 20, and automatically activate the stabilizer wheels (moving the stabilizing wheels to the second position), when the servo steering motor should be activated and/or when the roll data indicate that the bicycle 20 is not in a stable condition.

Preferably, the intervention of the brake and/or rotatable handlebar is only small enough to be perceptible by the user 22, but without creating of safety problems for the user 22. In fact, in the solutions described before, the user 12 does not directly remotely control the movement of the bicycle 20 (acceleration, braking, and/or steering), but only intervenes in the assistance system provided by the electric bicycle 20 to the user 22, reducing or increasing the assistance provided by the bicycle 20.

However, in various embodiments, the control circuit 70 may also control the bicycle 20 directly as a function of the data received from the stationary bike 10. For example, the processing circuit 700 may vary the speed of bicycle 20 as a function of the rotation speed of the pedals of stationary bike 10, e.g., drive motor 730 such that the speed of bicycle 20 is proportional to the rotation speed of the pedals of stationary bike 10. Similarly, the processing circuit 700 may actuate the motor 730 or directly a brake of the bicycle 20 to brake the bicycle 20 as a function of the actuation state of the lever and/or the force applied to the lever of stationary bike 10. Finally, the control circuit 70 may turn the handlebar of the bicycle 20 as a function of the rotation of the handlebar of stationary bike 10, e.g., drive a motor 730 such that the handlebar of the bicycle 20 is rotated to an angle that corresponds to the rotation angle of the handlebar of stationary bike 10. In this case, the processing circuit 700 may pre-process the received data, for example, to keep the absolute value and/or relative change of the speed of the bicycle 20 (for the pedaling or braking speed) or the rotation of the handlebar of the bicycle 20 within given predetermined limits.

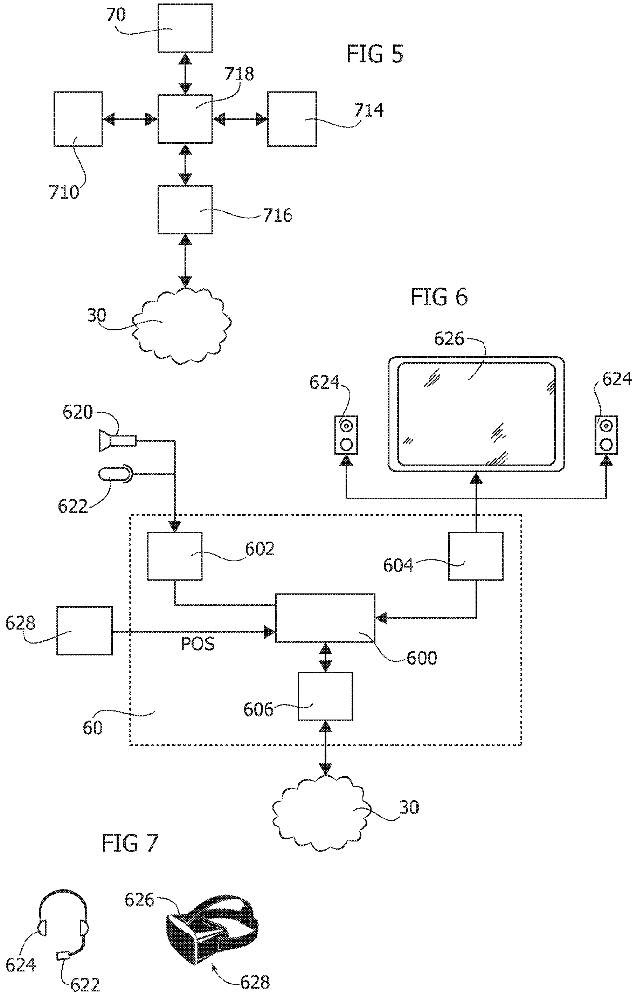

As shown in Figure 5, in various embodiments, the bicycle 20 may use a known control circuit 70 (without remote interaction with stationary bike 10). Specifically, in this case, the stationary bike 20 may include an additional processing circuit 718, such as a microprocessor programmed via software code, which is connected between the controls 714 and the control circuit 70. Such processing circuit 718 is configured to exchange via the communication interface 716 data with the stationary bike 10. Therefore, in various embodiments, the user 22 may set via the controls 714 a given level of assistance. However, such a signal is provided to processing circuit 718, and processing circuit 718 is configured to receive from stationary bike 10 data indicating the pedaling speed or pedaling torque (or similar data), and possibly the brake lever actuation, and add to the set assistance level a value being proportional to the pedaling speed or pedaling torque received from stationary bike 10. Similarly, the processing circuit 718 may subtract a predetermined value when the data indicate that the lever has been actuated (in case the stationary bike 10 provides only the actuation state of the brake lever) or subtract a value being proportional to the force applied to the brake lever. Accordingly, in this case, the processing circuit 718 emulates the operation of the controls 714 and provides to the control circuit 70 a modified signal. In general, it is sufficient that the signal is compatible with the control circuit 70, and may correspond to an analog signal or a digital signal in accordance with any communication protocol.

Similarly, the processing circuit 718 may monitor signals provided by the sensors 710, or additional signals provided by additional sensors 710' that provide similar signals, such as a sensor 710' configured to detect a rotation speed of the pedals 206 or one of the wheels 202/204.

Thus, in various embodiments. the components 718 and 716, and possibly the sensors 710', may be provided as an additional installation kit for a conventional electric bicycle.

In general, the data exchanged between the stationary bike 10 and bicycle 20 may be used to implement additional actuators as well. For example, in various embodiments, the stationary bike 10 may have associated an airflow generator, such as a fan, configured to generate an airflow proportional to the actual forward speed of the bicycle 20 or the pedaling speed of the bicycle 20, thereby better simulating cycling.

In various embodiments, one or more of the data exchanged between the processing circuits 500 and 700 (or 718) are filtered before or after data transmission, preferably by a (analog and/or digital) low-pass filter. For example, small variations in the data indicating the effort generated by the user 12 and/or user 22 can be filtered out in this way.

As explained before, in addition to or as an alternative to the interaction between the stationary bike 10 and the bicycle 20, a communication system may be provided to create an interaction between the users 12 and 22.

For example, Figure 6 shows an embodiment, in which the stationary bike 10 has associated an audio and/or video streaming system.

For this purpose, the system includes a microphone 622 configured to capture the voice of the user 12 using the bicycle 10. As mentioned before, optionally, the system may also include a camera 620 configured to capture images of the face of the user 12. Such a camera 620 is purely optional. For example, the microphone 622 and the camera 620 may be integrated into a webcam, e.g., having a USB connection. For example, the camera 620 may be mounted on the handlebar 104. Alternatively, the camera 620, with or without microphone 622, may be placed on the end of a bar that is in turn connected to a lightweight helmet worn by the user, thus making it fixed with respect to the face when the user turns his or her head.

The system also includes one or more screens 626 to display images received from the remote bicycle 20 or, in the case of a virtual viewpoint, generated as a function of images received in real time from the remote bicycle 20. The screen 626 may be flat, curved, or immersive, and may have any size, aspect ratio, resolution, response time, and be based on any technology. Finally, the system also includes speakers 624 to play audio data received from remote bicycle 20.

Accordingly, by using the microphone 622 and speakers 624, the user 12 can communicate with the user 22. In addition, the screen(s) 626 may show the path followed by the user 22, possibly calculating images that reflect the viewpoint of the user 12. For example, for this purpose, the system may include a sensor 628 configured to provide data POS indicative of the orientation of the head of the user 12 and/or the eyes of the user 12. For example, such sensors 628 may include a gyroscope and/or an acceleration sensor mounted on the head of the user 12, and/or an oculometry device, i.e., a so-called eye-tracker, and/or a camera that captures images of the head of the user 12. For example, such a camera may also correspond to the camera 620.

In the embodiment considered. a control circuit 60 then receives the audio data acquired by the microphone 622, and possibly the images acquired by the camera 620 and/or the position POS provided by the sensor 628. For example, in the embodiment considered, the control circuit 60 includes a processing circuit 600, for example comprising one or more microprocessors programmed by software code.

Thus, the processing circuit 600 may communicate with the microphone 622 and possibly the camera 620 via an interface circuit 602, such as a USB port. Optionally, the processing circuit 600 may process the images provided by the camera 620 to determine the instantaneous orientation POS of the head of the user 12, thereby implementing the sensor 628. Alternatively, an additional sensor 628 may be used.

In the embodiment considered, the processing circuit 600 then sends audio data and possibly video data and/or the position POS to the bicycle 20. For this purpose, the control circuit 60 includes a communication interface 606 to communicate via the network 30 with the remote bicycle 20. The communication interface 606 may have the same characteristics as the communication interface 508. Therefore, the control circuit 60 may be implemented with a conventional computer, such as a desktop computer or a laptop. In general, the control circuits 50 and 60 may also be combined. For example, the processing circuits 500 and 600 may be implemented with the same processing circuit and/or the communication interfaces 508 and 606 may be implemented with the same communication interface.

In parallel, the processing circuit 600 receives via the communication interface 606 audio and video data from the remote bicycle 20. Then the processing circuit 600 processes such audio and video data, e.g., to decode and/or decompress the data, and sends the processed data to the speakers 624 and the screen 624. For example, for this purpose, the control circuit 60 may include an interface circuit 604 to communicate with the speakers 624 and the screen(s) 624. For example, the interface 604 may include a sound card and a video card, for example implemented with an HDMI interface. Preferably, the sound card is multichannel, such as stereo or Dolby surround.

In particular, in case the remote bicycle 20 includes a rotatable camera, the processing circuit 600 may send the position POS to the remote bicycle 20, such that the remote bicycle 20 can rotate the respective camera as a function of the position POS. Therefore, in this case, the processing circuit 600 receives a single video stream that already corresponds to the orientation of the position of the user 12.

In contrast, in case the remote bicycle 20 includes a camera that takes panoramic images or a plurality of cameras with different orientations, the processing circuit 600 may send the position POS, such that the bicycle 20 can only return a single video stream that already corresponds to the orientation POS, thus implementing a camera that is virtually rotated. Alternatively, the remote bicycle 20 may send the panoramic images or video streams of the plurality of cameras, and the processing circuit 600 may determine the video stream to be shown on the screen 626 by an analysis of the user's virtual viewpoint, as indicated via the data POS. The person skilled in the art will appreciate that the former solution requires the transmission of less data, while the latter solution may be advantageous in permitting a smoother and more responsive change of the virtual viewpoint. Therefore, regardless of the specific implementation, the image shown on the screen 626 shows the path followed by the remote bicycle 20 in real time. In general, the position POS is purely optional, because the camera mounted on the remote bicycle 20 could also provide only a single video stream with static orientation, or for each screen 626 a respective video stream with static orientation (in case of multiple screens 626).

In general, as shown in Figure 7, the microphone 622 and speakers 624 may be integrated into a headset. Similarly, the screen(s) 626 may be integrated into a VR viewer, which is particularly useful when using a virtual viewpoint for the user 12, since VR viewers often already include sensors 628 that provide data indicating the orientation of the head of the user 12 and/or the eyes of the user 12. In general, the headset and viewer VR may also be integrated into a single device.

In various embodiments, the user interface 106 may include a button to change the mode of operation. In particular, in various embodiments, the remote bicycle 20 may include two cameras. A first (front) camera that captures the path followed by the bicycle 20, and a second (rear) camera that captures the face of the user 22. Therefore, the processing circuit 600 may be configured to send data indicating the mode of operation, and the remote bicycle 20 may return either the video stream capturing the path or the video stream capturing the face of the user 22. Therefore, in the first mode, the user 22 can pedal while observing the path followed by the bicycle 20 on the screen 626. In contrast, in the second mode, the user may essentially make a video call with the user 22. As will be described later, preferably the bicycle 20 does not show the video of the user 12 so as to not distract the user 22. However, the bicycle 20 may include a screen to show an avatar, possibly by synchronizing the avatar's facial movement with the voice of the user 12 or the facial movement of the user 12. In various embodiments, the switching between the modes of operation may also be operated in other ways, such as by other buttons, e.g., provided laterally on a viewer VR, by voice commands recorded via the microphone 622, and/or gestures that may be detected via the camera 620.

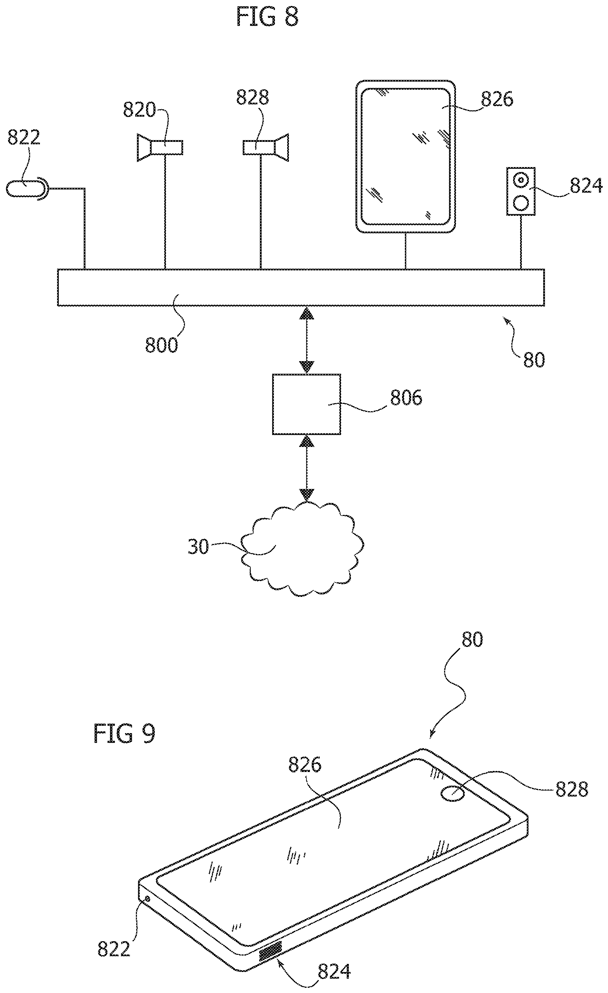

Finally, Figure 8 shows an embodiment, in which the bicycle 20 has associated an audio and/or video streaming device 80.

In the embodiment considered, the device 80 includes a microphone 822 configured to record the voice of the user 22 using the bicycle 20, speakers 824 to play audio data received from the processing circuit 600, and at least one camera. Specifically, the device 80 includes at least one camera 828 configured to capture the path followed by bicycle 20. As mentioned above, optionally, the device 80 may also include a second camera 820 configured to capture images of the face of the user 22. Such a camera 820 is purely optional.

In the embodiment considered, the microphone 822, the speakers 824, the camera(s) 828, and the optional camera 820 are connected to a processing circuit 800, such as a microprocessor programmed via software code. In the embodiment considered, the processing circuit 800 is connected to the network 30 via a communication interface 806, such as a mobile communication interface.

Accordingly, by using the microphone 822 and speakers 824, the user 22 may communicate with user 12. In various embodiments, the microphone 822 and the speakers 824 may also be implemented with a headset, such as a Bluetooth^{®} headset.

In various embodiments, the device 80 also includes a screen 826. In general, the screen 826 may be used to display an image received from the processing circuit 600 of the stationary bike 10, for example comprising the face of the user 12, or preferably an avatar of the face of the user 12. In general, such an avatar may be generated in the processing circuit 600, i.e., prior to transmission, or in the processing circuit 800, for example, depending on the audio and/or video data received from the processing circuit 600.

Accordingly, as shown in Figure 9, the device 80 in Figure 8 may be implemented with a smartphone or tablet, comprising a microphone 822 and speakers 824 (and/or a Bluetooth^{®} headset transceiver), a screen 826, a front camera 828 on the side of the screen 826, and a rear camera 820 on the opposite side of the screen 826 (not shown in Figure 9).

In various embodiments, the microphone 822, speakers 824, optional screen 826, and optional camera 820 may also be implemented in a device 80 with a respective communication interface 806, such as a mobile phone, and:
- the camera 828 may be connected via a wired or a wireless connection to such a device 80; or
- the camera 828 may be integrated into an additional device with a respective communication interface 806.

Moreover, as mentioned before, some of the functions of the processing circuit 700 may also be implemented in the processing circuit 800. For example, the processing circuit 800 may implement the processing circuit 714 that generates signals for the control circuit 70. Similarly, the processing circuit 800 may acquire one or more of the signals/data provided by the sensors 710 and/or 710'. For example, in various embodiments, the device 80 may include the satellite navigation receiver mentioned before, and the actuator 108 of the stationary bike may be driven as a function of the positions detected by that satellite navigation receiver, for example, as a function of a speed and possibly an inclination determined as a function of these position data. In general, the calculation of the speed or inclination, may be done in the processing circuit 800 or the processing circuit 500.

Accordingly, in the simplest case, the camera 828, and possibly the entire device 80, is mounted in a fixed/static position relative to the bicycle 20, such as on a support attached to the handlebar of the bicycle 20, where the camera 828 faces the front direction of the bicycle and the optional camera 820 faces the user 22. In general, the camera 828 may also be mounted on a support with optical stabilization.

In contrast, in other embodiments, the camera 828, and possibly the entire device 80, is mounted on a horizontally pivoting support, thus implementing a pan camera 828. For example, as mentioned before, in this case, the processing circuit 800 may receive orientation data POS and turn the camera 828 as a function of this data. For example, in various embodiments, the rotating support may be implemented with a motorized gimbal, e.g., having 2 or 3 degrees of freedom. Therefore, in various embodiments, the camera 828 may be a motorized pan and tilt camera, i.e., a so-called pan-tilt camera. For example, in various embodiments, the microphone 822, camera 828, and speakers 824 are integrated into a robotic head, e.g., with anthropomorphic features. In this case, the camera 828 may be a stereo-camera arranged in correspondence of the two eyes, with human-like field of view. Similarly, the microphone 822 may be implemented with two microphones at the robot's ears and a speaker may be arranged at the mouth. For example, in this case, one or more actuators associated with the robotic head may be driven as a function of the position of the head of the user 12, the facial movement of the user 12, and/or the voice of the user 12, thereby essentially generating a mechanical avatar that emulates the behavior of the user 12.

In various embodiments, the camera 828 may be a panoramic camera or comprise a plurality of cameras, such that the camera 828 has a field of view between 135° and 360°, for example, between 150° and 270°. In this case:
- the camera 828 may send the image(s) to the processing circuit 600, and the processing circuit 600 may generate the images to be shown on a plurality of screens 626;
- the camera 828 may send the image or images to the processing circuit 600, and the processing circuit 600 may generate the image to be shown on the screen 626 as a function of the position POS; or
- the processing circuit 800 may extract a part of the panoramic image or generate an image via a virtual viewpoint analysis depending on the position POS and send only the extracted/generated image to the processing circuit 600.

Of course, without prejudice to the underlying principles of the invention, the details of construction and embodiments may vary widely from what has been described and illustrated herein purely by way of example, without thereby departing from the scope of the present invention as defined by the claims.

## Claims

1. A system for physical activity, comprising:
- a stationary bike (10) comprising an actuator (108) configured to set a pedaling resistance of said stationary bike (10);
- a bicycle (20) comprising at least two wheels (202, 204);
wherein said stationary bike (10) comprises a first communication interface (506, 606) and said bicycle (20) comprises a second communication interface (706, 806), wherein said first communication interface (506, 606) and said second communication interface (706, 806) are configured to exchange data via a Wide-Area Network (30);
wherein said stationary bike (10) comprises a first microphone (622) configured to record the voice of a first user (12) using said stationary bike (10), a first speaker (624), a first display (626), and a first processing circuit (500, 600) configured to:
- record a first audio signal by means of said first microphone (622) and transmit said first audio signal via said first communication interface (506, 606) to said second communication interface (706, 806),
- receive a second audio signal via said first communication interface (506, 606) from said second communication interface (706, 806) and reproduce said second audio signal by means of said first speaker (624),
- receive a first sequence of images by means of said first communication interface (506, 606) from said second communication interface (706, 806) and show said first sequence of images by means of said first display (626);
wherein said bicycle (20) comprises a second microphone (822) configured to record the voice of a second user (22) using said bicycle (20), a second speaker (824), a first camera (828) configured to record a route followed by said bicycle (20), and a second processing circuit (700, 800) configured to:
- receive said first audio signal via said second communication interface (706, 806) from said first communication interface (506, 606) and reproduce said first audio signal by means of said second speaker (824),
- record said second audio signal by means of said second microphone (822) and transmit said second audio signal via said second communication interface (706, 806) to said first communication interface (506, 606),
- record said first sequence of images by means of said first camera (828) and transmit said first sequence of images via said second communication interface (706, 806) to said first communication interface (506, 606);
wherein said bicycle comprises a first sensor (710; 710') configured to detect first data indicative of the effort generated by said second user (22) or to be generated by said second user (22), wherein said first sensor (710; 710') is configured to determine the speed of the bicycle (20), the torque applied to the pedals (206) of the bicycle (20), and/or the inclination of the bicycle (20);
wherein said second processing circuit (700, 800) is configured to transmit said first data to said first processing circuit (500, 600);
wherein said first processing circuit (500, 600) is configured to drive (504) said actuator (108) to vary said pedaling resistance of said stationary bike (10) as a function of said first data in order to increase the effort of said first user (12) when the effort of said second user increases (22) and decrease the effort of said first user (12) when the effort of said second user decreases (22);
**characterized in that**
said stationary bike (10) comprises a second sensor (502) configured to detect second data indicative of the speed of rotation of the pedals (102) of said stationary bike (10) and/or the torque applied to the pedals (102) of said stationary bike (10);
wherein said first processing circuit (500, 600) is configured to transmit said second data to said second processing circuit (700, 800);
wherein said bicycle (20) is a bicycle with electrically assisted pedaling; and
wherein said second processing circuit (700, 800) is configured to vary the level of assistance provided by said bicycle with electrically assisted pedaling as a function of said second data in order to increase the level of assistance when the speed of rotation of the pedals (102) of said stationary bike (10) and/or the torque applied to the pedals (102) of said stationary bike (10) increase and decrease the level of assistance when the speed of rotation of the pedals (102) of said stationary bike and/or the torque applied to the pedals (102) of said stationary bike decrease.

2. The system according to Claim 1,
wherein said stationary bike (10) comprises a sensor to detect the operating status of a lever and/or the force applied to said lever,
wherein said first processing circuit (500, 600) is configured to transmit the drive state of said lever and/or the force applied to said lever to said second processing circuit (700, 800),
wherein said second processing circuit (700, 800) is configured to vary the level of assistance provided by said electrically assisted bicycle according to the operating state of said lever and/or the force applied to said lever in order to reduce the level of assistance when said lever is operated.

3. The system according to any of the previous claims,
wherein said stationary bike (10) comprises a third sensor configured to detect third data indicative of the rotation of a handlebar of said stationary bike (10),
wherein said first processing circuit (500, 600) is configured to transmit said third data to said second processing circuit (700, 800),
wherein said bicycle (20) comprises a handlebar having associated a servo-steering, and
wherein said second processing circuit (700, 800) is configured to drive said servo-steering according to said third data.

4. The system according to any of the previous claims, wherein said stationary bike (10) comprises a position sensor (628) configured to detect the position (POS) of the head of said first user (12) and/or the eyes of said first user (12), and
- said first camera (828) is a camera configured to acquire panoramic images or said first camera (828) comprises a plurality of cameras configured to acquire a plurality of images with different orientations with respect to said bicycle (20), and wherein said first processing circuit (500, 600) and said second processing circuit (700, 800) are configured to record by means of said first camera (828) a first sequence of said panoramic images or of said plurality of images with different orientations, and generate said first sequence of images according to said position (POS) and said first sequence of said panoramic images or said plurality of images with different orientations; or
- said first camera (828) is a rotating camera, and said second processing circuit (700, 800) is configured to rotate said first camera (828) according to said position (POS).

5. The system according to Claim 4, wherein said first display (626) and said position sensor (628) are integrated into a virtual reality viewer.

6. The system according to any of the previous claims, wherein said stationary bike (10) comprises a second camera (820) and said bicycle (20) comprises a second display (826), and wherein said first processing circuit (500, 600) and said second processing circuit (700, 800) are configured to:
- record a second sequence of images using the second camera (820);
- generate an avatar of the face of said first user (12) as a function of said second sequence of images; and
- show said avatar by means of said second display (826).

7. The system according to any of the previous claims, wherein said electric bicycle (20) includes a motor (730) and controls (714) to set a level of assistance to be generated by said electric motor (730), and wherein said second processing circuit (700, 800) is configured to vary said level of assistance as a function of said second data.

8. The system according to Claim 7, wherein said second processing circuit (700, 800) is configured to:
- scale a signal provided by said controls (714) as a function of said second data; or
- add to said level of assistance a value being proportional to said second data.

9. A method of operating a system according to any of the previous claims, comprising:
- recording a first audio signal by means of said first microphone (622) of said stationary bike (10) and reproducing said first audio signal by means of said second speaker (824) of said bicycle (20),
- recording said second audio signal by means of said second microphone (822) of said bicycle (20) and reproducing said second audio signal by means of said first speaker (624) of said stationary bike (10),
- recording said first sequence of images by means of said first camera (828) of said bicycle (20) and showing said first sequence of images by means of said first display (626) of said stationary bike (10),
- detecting via said first sensor (710; 710') said first data and driving (504) said actuator (108) to vary said pedaling resistance of said stationary bike (10) as a function of said first data in order to increase the effort of said first user (12) when the effort of said second user increases (22) and decrease the effort of said first user (12) when the effort of said second user decreases (22), wherein said first sensor (710; 710') is configured to determine the speed of the bicycle (20), the torque applied to the pedals (206) of the bicycle (20), and/or the inclination of the bicycle (20), and varying the level of assistance provided by said bicycle with electrically assisted pedaling as a function of said second data in order to increase the level of assistance when the speed of rotation of the pedals (102) of said stationary bike (10) and/or the torque applied to the pedals (102) of said stationary bike (10) increase and decrease the level of assistance when the speed of rotation of the pedals (102) of said stationary bike and/or the torque applied to the pedals (102) of said stationary bike decrease

## Patentansprüche

1. System für körperliche Aktivität, das Folgendes umfasst:
- ein Standrad (10), das einen Aktor (108) umfasst, der konfiguriert ist, einen Pedalwiderstand des Standrads (10) einzustellen;
- ein Fahrrad (20), das mindestens zwei Räder (202, 204) umfasst;
wobei das Standrad (10) eine erste Kommunikationsschnittstelle (506, 606) umfasst und das Fahrrad (20) eine zweite Kommunikationsschnittstelle (706, 806) umfasst, wobei die erste Kommunikationsschnittstelle (506, 606) und die zweite Kommunikationsschnittstelle (706, 806) konfiguriert sind, über ein Fernbereichsnetz (30) Daten auszutauschen; wobei das Standrad (10) ein erstes Mikrofon (622), das konfiguriert ist, die Stimme eines ersten Benutzers (12) aufzuzeichnen, der das Standrad (10) verwendet, einen ersten Lautsprecher (624), eine erste Anzeigeeinheit (626) und eine erste Verarbeitungsschaltung (500, 600) umfasst, die konfiguriert ist zum:
- Aufzeichnen eines ersten Audiosignals mittels des ersten Mikrofons (622) und Senden des ersten Audiosignals über die erste Kommunikationsschnittstelle (506, 606) an die zweite Kommunikationsschnittstelle (706, 806),
- Empfangen eines zweiten Audiosignals über die erste Kommunikationsschnittstelle (506, 606) von der zweiten Kommunikationsschnittstelle (706, 806) und Wiedergeben des zweiten Audiosignals mittels des ersten Lautsprechers (624),
- Empfangen einer ersten Abfolge von Bildern mittels der ersten Kommunikationsschnittstelle (506, 606) von der zweiten Kommunikationsschnittstelle (706, 806) und Zeigen der ersten Abfolge von Bildern mittels der ersten Anzeigeeinheit (626);
wobei das Fahrrad (20) ein zweites Mikrofon (822), das konfiguriert ist, die Stimme eines zweiten Benutzers (22) aufzuzeichnen, der das Fahrrad (20) verwendet, einen zweiten Lautsprecher (824),
eine erste Kamera (828), die konfiguriert ist, einen Weg, dem das Fahrrad (20) folgt, aufzuzeichnen, und eine zweite Verarbeitungsschaltung (700, 800) umfasst, die konfiguriert ist zum:
- Empfangen des ersten Audiosignals über die zweite Kommunikationsschnittstelle (706, 806) von der ersten Kommunikationsschnittstelle (506, 606) und Wiedergeben des ersten Audiosignals mittels des zweiten Lautsprechers (824),
- Aufzeichnen des zweiten Audiosignals mittels des zweiten Mikrofons (822) und Senden des zweiten Audiosignals über die zweite Kommunikationsschnittstelle (706, 806) an die erste Kommunikationsschnittstelle (506, 606) ,
- Aufzeichnen der ersten Abfolge von Bildern mittels der ersten Kamera (828) und Senden der ersten Abfolge von Bildern über die zweite Kommunikationsschnittstelle (706, 806) an die erste Kommunikationsschnittstelle (506, 606) ;
wobei das Fahrrad einen ersten Sensor (710; 710') umfasst, der konfiguriert ist, erste Daten zu detektieren, die die Leistung angeben, die durch den zweiten Benutzer (22) erzeugt wird oder durch den zweiten Benutzer (22) erzeugt werden soll, wobei der erste Sensor (710; 710') konfiguriert ist, die Geschwindigkeit des Fahrrads (20), das Drehmoment, das auf die Pedale (206) des Fahrrads (20) ausgeübt wird, und/oder die Neigung des Fahrrads (20) zu bestimmen;
wobei die zweite Verarbeitungsschaltung (700, 800) konfiguriert ist, die ersten Daten an die erste Verarbeitungsschaltung (500, 600) zu senden;
wobei die erste Verarbeitungsschaltung (500, 600) konfiguriert ist, den Aktor (108) anzusteuern (504), den Pedalwiderstand des Standrads (10) als eine Funktion der ersten Daten zu verändern, um die Leistung des ersten Benutzers (12) zu erhöhen, wenn die Leistung des zweiten Benutzers (22) zunimmt, und die Leistung des ersten Benutzers (12) zu verringern, wenn die Leistung des zweiten Benutzers (22) abnimmt;
**dadurch gekennzeichnet, dass**
das Standrad (10) einen zweiten Sensor (502) umfasst, der konfiguriert ist, zweite Daten zu detektieren, die die Drehzahl der Pedale (102) des Standrads (10) und/oder das Drehmoment, das auf die Pedale (102) des Standrads (10) ausgeübt wird, angeben;
wobei die erste Verarbeitungsschaltung (500, 600) konfiguriert ist, die zweiten Daten an die zweite Verarbeitungsschaltung (700, 800) zu senden;
wobei das Fahrrad (20) ein Fahrrad mit elektrisch unterstütztem Treten in die Pedale ist; und
wobei die zweite Verarbeitungsschaltung (700, 800) konfiguriert ist, den Grad der Unterstützung, die durch das Fahrrad mit elektrisch unterstütztem Treten in die Pedale bereitgestellt wird, als eine Funktion der zweiten Daten zu verändern, um den Grad der Unterstützung zu erhöhen, wenn die Drehzahl der Pedale (102) des Standrads (10) und/oder das Drehmoment, das auf die Pedale (102) des Standrads (10) ausgeübt wird, zunehmen, und den Grad der Unterstützung zu verringern, wenn die Drehzahl der Pedale (102) des Standrads und/oder das Drehmoment, das auf die Pedale (102) des Standrads ausgeübt wird, abnehmen.

2. System nach Anspruch 1,
wobei das Standrad (10) einen Sensor, um den Betätigungszustand eines Hebels und/oder die Kraft, die auf den Hebel ausgeübt wird, zu detektieren, umfasst, wobei die erste Verarbeitungsschaltung (500, 600) konfiguriert ist, den Ansteuerzustand des Hebels und/oder die Kraft, die auf den Hebel ausgeübt wird, an die zweite Verarbeitungsschaltung (700, 800) zu senden,
wobei die zweite Verarbeitungsschaltung (700, 800) konfiguriert ist, den Grad der Unterstützung, die durch das elektrisch unterstützte Fahrrad bereitgestellt wird, gemäß dem Betätigungszustand des Hebels und/oder der Kraft, die auf den Hebel ausgeübt wird, zu verändern, um den Grad der Unterstützung zu verringern, wenn der Hebel betätigt wird.

3. System nach einem der vorhergehenden Ansprüche, wobei das Standrad (10) einen dritten Sensor umfasst, der konfiguriert ist, dritte Daten zu detektieren, die die Drehung einer Lenkstange des Standrads (10) angeben; wobei die erste Verarbeitungsschaltung (500, 600) konfiguriert ist, die dritten Daten an die zweite Verarbeitungsschaltung (700, 800) zu senden,
wobei das Fahrrad (20) eine Lenkstange, der eine Servolenkung zugeordnet ist, umfasst und
wobei die zweite Verarbeitungsschaltung (700, 800) konfiguriert ist, die Servolenkung gemäß den dritten Daten anzusteuern.

4. System nach einem der vorhergehenden Ansprüche, wobei das Standrad (10) einen Positionssensor (628) umfasst, der konfiguriert ist, die Position (POS) des Kopfs des ersten Benutzers (12) und/oder der Augen des ersten Benutzers (12) zu detektieren, und
- die erste Kamera (828) eine Kamera ist, die konfiguriert ist, Panoramabilder zu erfassen, oder die erste Kamera (828) mehrere Kameras umfasst, die konfiguriert sind, mehrere Bilder mit verschiedenen Orientierungen in Bezug auf das Fahrrad (20) zu erfassen, und wobei die erste Verarbeitungsschaltung (500, 600) und die zweite Verarbeitungsschaltung (700, 800) konfiguriert sind, mittels der ersten Kamera (828) eine erste Abfolge der Panoramabilder oder der mehreren Bilder mit verschiedenen Orientierungen aufzuzeichnen und die erste Abfolge von Bildern gemäß der Position (POS) und der ersten Abfolge von Panoramabildern oder der mehreren Bilder mit verschiedenen Orientierungen zu erzeugen; oder
- die erste Kamera (828) eine sich drehende Kamera ist und die zweite Verarbeitungsschaltung (700, 800) konfiguriert ist, die erste Kamera (828) gemäß der Position (POS) zu drehen.

5. System nach Anspruch 4, wobei die erste Anzeigeeinheit (626) und der Positionssensor (628) in einer Betrachtungseinheit für virtuelle Realität integriert sind.

6. System nach einem der vorhergehenden Ansprüche, wobei das Standrad (10) eine zweite Kamera (820) umfasst und das Fahrrad (20) eine zweite Anzeigeeinheit (826) umfasst, und wobei die erste Verarbeitungsschaltung (500, 600) und die zweite Verarbeitungsschaltung (700, 800) konfiguriert sind zum:
- Aufzeichnen einer zweiten Abfolge von Bildern unter Verwendung der zweiten Kamera (820);
- Erzeugen eines Avatars des Gesichts des ersten Benutzers (12) als eine Funktion der zweiten Abfolge von Bildern; und
- Zeigen des Avatars mittels der zweiten Anzeigeeinheit (826).

7. System nach einem der vorhergehenden Ansprüche, wobei das Elektrofahrrad (20) einen Motor (730) und Bedienungselemente (714), um einen Grad der Unterstützung, die durch den Elektromotor (730) erzeugt werden soll, einzustellen, enthält und wobei die zweite Verarbeitungsschaltung (700, 800) konfiguriert ist, den Grad der Unterstützung als eine Funktion der zweiten Daten zu verändern.

8. System nach Anspruch 7, wobei die zweite Verarbeitungsschaltung (700, 800) konfiguriert ist zum:
- Skalieren eines Signals, das durch die Bedienungselemente (714) bereitgestellt wird, als eine Funktion der zweiten Daten; oder
- Addieren eines Wertes, der zu den zweiten Daten proportional ist, zu dem Grad der Unterstützung.

9. Verfahren zum Betreiben eines Systems nach einem der vorhergehenden Ansprüche, das Folgendes umfasst:
- Aufzeichnen eines ersten Audiosignals mittels des ersten Mikrofons (622) des Standrads (10) und Wiedergeben des ersten Audiosignals mittels des zweiten Lautsprechers (824) des Fahrrads (20),
- Aufzeichnen des zweiten Audiosignals mittels des zweiten Mikrofons (822) des Fahrrads (20) und Wiedergeben des zweiten Audiosignals mittels des ersten Lautsprechers (624) des Standrads (10),
- Aufzeichnen der ersten Abfolge von Bildern mittels der ersten Kamera (828) des Fahrrads (20) und Zeigen der ersten Abfolge von Bildern mittels der ersten Anzeigeeinheit (626) des Standrads (10),
- Detektieren der ersten Daten über den ersten Sensor (710; 710') und Ansteuern (504) des Aktors (108), den Pedalwiderstand des Standrads (10) als eine Funktion der ersten Daten zu verändern, um die Leistung des ersten Benutzers (12) zu erhöhen, wenn die Leistung des zweiten Benutzers (22) zunimmt, und die Leistung des ersten Benutzers (12) zu verringern, wenn die Leistung des zweiten Benutzers (22) abnimmt, wobei der erste Sensor (710, 710') konfiguriert ist, die Geschwindigkeit des Fahrrads (20), das Drehmoment, das auf die Pedale (206) des Fahrrads (20) ausgeübt wird, und/oder die Neigung des Fahrrads (20) zu bestimmen, und Verändern des Grads der Unterstützung, die durch das Fahrrad mit elektrisch unterstütztem Treten in die Pedale bereitgestellt wird, als eine Funktion der zweiten Daten, um den Grad der Unterstützung zu erhöhen, wenn die Drehzahl der Pedale (102) des Standrads (10) und/oder das Drehmoment, das auf die Pedale (102) des Standrads (10) ausgeübt wird, zunehmen, und den Grad der Unterstützung zu verringern, wenn die Drehzahl der Pedale (102) des Standrads und/oder das Drehmoment, das auf die Pedale (102) des Standrads ausgeübt wird, abnehmen.

## Revendications

1. Système d'activité physique, comprenant :
- un vélo stationnaire (10) comprenant un actionneur (108) configuré pour définir une résistance de pédalage dudit vélo stationnaire (10) ;
- un vélo (20) comprenant au moins deux roues (202, 204) ;
ledit vélo stationnaire (10) comprenant une première interface de communication (506, 606) et ledit vélo (20) comprenant une deuxième interface de communication (706, 806), ladite première interface de communication (506, 606) et ladite deuxième interface de communication (706, 806) étant configurées pour échanger des données via un réseau étendu (30) ;
ledit vélo stationnaire (10) comprenant un premier microphone (622) configuré pour enregistrer la voix d'un premier utilisateur (12) en utilisant ledit vélo stationnaire (10), un premier haut-parleur (624), un premier affichage (626), et un premier circuit de traitement (500, 600) configuré pour :
- enregistrer un premier signal audio au moyen dudit premier microphone (622) et transmettre ledit premier signal audio via ladite première interface de communication (506, 606) à ladite deuxième interface de communication (706, 806),
- recevoir un deuxième signal audio via ladite première interface de communication (506, 606) en provenance de ladite deuxième interface de communication (706, 806) et reproduire ledit deuxième signal audio au moyen dudit premier haut-parleur (624),
- recevoir une première séquence d'images au moyen de ladite première interface de communication (506, 606) en provenance de ladite deuxième interface de communication (706, 806) et représenter ladite première séquence d'images au moyen dudit premier affichage (626) ;
ledit vélo (20) comprenant un deuxième microphone (822) configuré pour enregistrer la voix d'un deuxième utilisateur (22) en utilisant ledit vélo (20), un deuxième haut-parleur (824),
une première caméra (828) configurée pour enregistrer un trajet suivi par ledit vélo (20), et un deuxième circuit de traitement (700, 800) configuré pour :
- recevoir ledit premier signal audio via ladite deuxième interface de communication (706, 806) en provenance de ladite première interface de communication (506, 606) et reproduire ledit premier signal audio au moyen dudit deuxième haut-parleur (824),
- enregistrer ledit deuxième signal audio au moyen dudit deuxième microphone (822) et transmettre ledit deuxième signal audio via ladite deuxième interface de communication (706, 806) à ladite première interface de communication (506, 606),
- enregistrer ladite première séquence d'images au moyen de ladite première caméra (828) et transmettre ladite première séquence d'images via ladite deuxième interface de communication (706, 806) à ladite première interface de communication (506, 606) ;
ledit vélo comprenant un premier capteur (710 ; 710') configuré pour détecter des premières données représentant l'effort généré par ledit deuxième utilisateur (22) ou à générer par ledit deuxième utilisateur (22), ledit premier capteur (710 ; 710') étant configuré pour déterminer la vitesse du vélo (20), le couple appliqué aux pédales (206) du vélo (20), et/ou l'inclinaison du vélo (20) ;
ledit deuxième circuit de traitement (700, 800) étant configuré pour transmettre lesdites premières données audit premier circuit de traitement (500, 600) ;
ledit premier circuit de traitement (500, 600) étant configuré pour entraîner (504) ledit actionneur (108) pour faire varier ladite résistance de pédalage dudit vélo stationnaire (10) en fonction desdites premières données afin d'augmenter l'effort dudit premier utilisateur (12) lorsque l'effort dudit deuxième utilisateur (22) augmente et diminuer l'effort dudit premier utilisateur (12) lorsque l'effort dudit deuxième utilisateur (22) diminue ;
**caractérisé en ce que**
ledit vélo stationnaire (10) comprend un deuxième capteur (502) configuré pour détecter des deuxièmes données représentant la vitesse de rotation des pédales (102) dudit vélo stationnaire (10) et/ou le couple appliqué aux pédales (102) dudit vélo stationnaire (10) ;
ledit premier circuit de traitement (500, 600) étant configuré pour transmettre lesdites deuxièmes données audit deuxième circuit de traitement (700, 800) ;
ledit vélo (20) étant un vélo à pédalage à assistance électrique ; et
ledit deuxième circuit de traitement (700, 800) étant configuré pour faire varier le niveau d'assistance fournie par ledit vélo à pédalage à assistance électrique en fonction desdites deuxièmes données afin d'augmenter le niveau d'assistance lorsque la vitesse de rotation des pédales (102) dudit vélo stationnaire (10) et/ou le couple appliqué aux pédales (102) dudit vélo stationnaire (10) augmentent et diminuer le niveau d'assistance lorsque la vitesse de rotation des pédales (102) dudit vélo stationnaire et/ou le couple appliqué aux pédales (102) dudit vélo stationnaire diminuent.

2. Système selon la revendication 1,
ledit vélo stationnaire (10) comprenant un capteur pour détecter l'état de fonctionnement d'un levier et/ou la force appliquée audit levier,
ledit premier circuit de traitement (500, 600) étant configuré pour transmettre l'état d'entraînement dudit levier et/ou la force appliquée audit levier audit deuxième circuit de traitement (700, 800),
ledit deuxième circuit de traitement (700, 800) étant configuré pour faire varier le niveau d'assistance fournie par ledit vélo à assistance électrique en fonction de l'état de fonctionnement dudit levier et/ou de la force appliquée audit levier pour réduire le niveau d'assistance lorsque ledit levier est actionné.

3. Système selon l'une quelconque des revendications précédentes,
ledit vélo stationnaire (10) comprenant un troisième capteur configuré pour détecter des troisièmes données représentant la rotation d'un guidon dudit vélo stationnaire (10),
ledit premier circuit de traitement (500, 600) étant configuré pour transmettre lesdites troisièmes données audit deuxième circuit de traitement (700, 800),
ledit vélo (20) comprenant un guidon ayant une direction assistée associée, et
ledit deuxième circuit de traitement (700, 800) étant configuré pour entraîner ladite direction assistée en fonction desdites troisièmes données.

4. Système selon l'une quelconque des revendications précédentes, ledit vélo stationnaire (10) comprenant un capteur de position (628) configuré pour détecter la position (POS) de la tête dudit premier utilisateur (12) et/ou les yeux dudit premier utilisateur (12), et
- ladite première caméra (828) étant une caméra configurée pour acquérir des images panoramiques ou ladite première caméra (828) comprenant une pluralité de caméras configurées pour acquérir une pluralité d'images avec des orientations différentes par rapport audit vélo (20), et ledit premier circuit de traitement (500, 600) et ledit deuxième circuit de traitement (700, 800) étant configurés pour enregistrer au moyen de ladite première caméra (828) une première séquence desdites images panoramiques ou de ladite pluralité d'images avec des orientations différentes, et générer ladite première séquence d'images selon ladite position (POS) et ladite première séquence desdites images panoramiques ou de ladite pluralité d'images avec des orientations différentes ; ou
- ladite première caméra (828) étant une caméra rotative, et ledit deuxième circuit de traitement (700, 800) étant configuré pour faire tourner ladite première caméra (828) selon ladite position (POS).

5. Système selon la revendication 4, ledit premier affichage (626) et ledit capteur de position (628) étant intégrés dans un visualiseur de réalité virtuelle.

6. Système selon l'une quelconque des revendications précédentes, ledit vélo stationnaire (10) comprenant une deuxième caméra (820) et ledit vélo (20) comprenant un deuxième affichage (826), et ledit premier circuit de traitement (500, 600) et ledit deuxième circuit de traitement (700, 800) étant configurés pour :
- enregistrer une deuxième séquence d'images en utilisant la deuxième caméra (820) ;
- générer un avatar du visage dudit premier utilisateur (12) en fonction de ladite deuxième séquence d'images ; et
- montrer ledit avatar au moyen dudit deuxième affichage (826).

7. Système selon l'une quelconque des revendications précédentes, ledit vélo (20) électrique comportant un moteur (730) et des commandes (714) pour définir un niveau d'assistance à générer par ledit moteur (730) électrique, et ledit deuxième circuit de traitement (700, 800) étant configuré pour faire varier ledit niveau d'assistance en fonction desdites deuxièmes données.

8. Système selon la revendication 7, ledit deuxième circuit de traitement (700, 800) étant configuré pour :
- mettre à l'échelle un signal fourni par lesdites commandes (714) en fonction desdites deuxièmes données ; ou
- ajouter audit niveau d'assistance une valeur proportionnelle auxdites deuxièmes données.

9. Procédé de fonctionnement d'un système selon l'une quelconque des revendications précédentes, comprenant :
- l'enregistrement d'un premier signal audio au moyen dudit premier microphone (622) dudit vélo stationnaire (10) et la reproduction dudit premier signal audio au moyen dudit deuxième haut-parleur (824) dudit vélo (20),
- l'enregistrement dudit deuxième signal audio au moyen dudit deuxième microphone (822) dudit vélo (20) et la reproduction dudit deuxième signal audio au moyen dudit premier haut-parleur (624) dudit vélo stationnaire (10),
- l'enregistrement de ladite première séquence d'images au moyen de ladite première caméra (828) dudit vélo (20) et la présentation de ladite première séquence d'images au moyen dudit premier affichage (626) dudit vélo stationnaire (10),
- la détection via ledit premier capteur (710 ; 710') desdites premières données et l'entraînement (504) dudit actionneur (108) pour faire varier ladite résistance de pédalage dudit vélo stationnaire (10) en fonction desdites premières données afin d'augmenter l'effort dudit premier utilisateur (12) lorsque l'effort dudit deuxième utilisateur (22) augmente et diminuer l'effort dudit premier utilisateur (12) lorsque l'effort dudit deuxième utilisateur (22) diminue, ledit premier capteur (710 ; 710') étant configuré pour déterminer la vitesse du vélo (20), le couple appliqué aux pédales (206) du vélo (20) et/ou l'inclinaison du vélo (20), et la variation du niveau d'assistance fournie par ledit vélo à pédale à assistance électrique en fonction desdites deuxièmes données afin d'augmenter le niveau d'assistance lorsque la vitesse de rotation des pédales (102) dudit vélo stationnaire (10) et/ou le couple appliqué aux pédales (102) dudit vélo stationnaire (10) augmentent et diminuer le niveau d'assistance lorsque la vitesse de rotation des pédales (102) dudit vélo stationnaire et/ou le couple appliqué aux pédales (102) dudit vélo stationnaire diminuent.
